# EUROPÄISCHE PATENTANMELDUNG

(11) **EP 2 098 241 A1**
(43) Veröffentlichungstag der Anmeldung: **09.09.2009**
(21) Anmeldenummer: 09001864.9
(22) Anmeldetag: 11.02.2009
(51) Int. Cl.: A61K 36/28, A61K 36/53, A61K 36/282, A61K 36/484, A61K 36/515, A61P 1/00, A61P 3/08, A61P 3/14, A23K 1/18, A61K 31/70, A61K 33/14

(54) **Verfahren zur vorbeugenden Behandlung von Gesundheitsstörungen bei kalbenden Kühen**

(30) Priorität: 18.02.2008 DE 102008009800
(71) Anmelder: Andresen, Uwe, 25767 Albersdorf (DE)
(72) Erfinder: Andresen, Uwe, 25767 Albersdorf (DE)
(74) Vertreter: Bünemann, Egon

(57) **Zusammenfassung**

Die Erfindung betrifft ein Verfahren zur vorbeugenden Behandlung von Gesundheitsstörungen bei kalbenden Kühen, bei dem den Kühen eine Wirkstoffmischung enthaltenes Wasser zum Trinken angeboten wird, wobei die Wirkstoffmischung wenigstens einen trinkanregenden, natürlichen oder naturidentischen Geschmacksstoff enthält, der einer chemischen Gruppe außerhalb der Gruppe der Zucker zugeordnet ist.

## Beschreibung

Die Erfindung betrifft ein Verfahren zur vorbeugenden Behandlung von Gesundheitsstörungen bei kalbenden Kühen.

Die gesundheitlichen Hauptprobleme in der modernen, milchmengenleistungsorientierten Kuhhaltung liegen in der Zeit vor und unmittelbar nach dem Abkalben. Lehrbuch bekannt spielen neben der Hypocalcaemie (Milchfieber), Ketosen und Labmagenverlagerungen die größte Rolle.

Die Hypocalcaemie führt typischerweise zum sog. Festliegen, d.h. zum Unvermögen der Kühe aufzustehen. Die ungenügende Adaption an die unmittelbar nach der Geburt einsetzende starke Milchproduktion führt zu erheblichen Verlusten an Calciumsalzen, die wiederum zu einer Lähmung der Muskulatur führen. Zur Vorbeugung werden den kalbenden Kühen zwangsweise Calciumsalze in Form von Gelen oder Tabletten oral mit speziellen Geräten verabreicht. Diese Zwangsverabreichung ist mit einem erheblichen Kraftaufwand seitens der eingebenden Person und oft mit Angst- und Schmerzzuständen der Tiere verbunden.

Bei der Labmagenverlagerung schiebt sich der Drüsenmagen unter dem Pansen und verlagert sich auf die rechte oder die linke Körperseite zwischen Pansen und Körperwand. Ursache dieser Labmagenverlagerung ist eine mangelnde Pansenfülle zum Zeitpunkt der Geburt des Kalbes und der Verlust der mechanischen Barrierewirkung des gefüllten Pansens zur Verhinderung einer derartigen Verlagerung. Hinzu kommt der raumschaffende Abgang des Kalbes und des Fruchtwassers während der Geburt. Zudem kommt es während der Geburtsphase zu einem vermehrten Kotabsatz, der zu einer weiteren Entleerung der Bauchhöhle führt. Auch nimmt die kalbende Kuh am Tag der Geburt kaum Nahrungsmittel und Trinkwasser auf, während gesunde, nicht kalbende Kühe bis zu 80 Liter Trinkwasser pro Tag aufnehmen. Neben der häufig erfolglosen Methode des Wälzens der Kuh, kommt als Behandlungsmaßnahme zur Reposition des verlagerten Labmagens lediglich die Operation und Fixierung in Frage.

Als vorbeugende Maßnahme zur Verhinderung der Verlagerung wird den kalbenden Kühen mit speziellen Pumpsystemen 50 bis 60 Liter Wasser über einen Schlauch direkt in den Pansen gefüllt. Der Nachteil dieser Methode besteht jedoch darin, dass sie erheblichen Kraftaufwand seitens der behandelnden Person erfordert und bis zu 10 Prozent der so behandelten Tiere als deren Folgen Lungenentzündungen davontragen.

Als Ketose wird eine Stoffwechselstörung der Milchkühe bezeichnet bei der es in den ersten Wochen nach der Geburt durch die plötzlich einsetzende Milchleistung zu einem Energiedefizit des Organismus kommt. Dieser wird verstärkt durch mangelnden Appetit nach der Geburt und die begrenzte Möglichkeit hochkonzentrierter Energiezufuhr durch eine zwingend erforderliche wiederkauergerechte Fütterung.
Als Folge des nahrungsbedingten Energiedefizits kommt es zu einem massiven Abbau von Körperfett der Kühe. Dabei entstehen Stoffwechselprodukte wie z.B. Aceton und andere so genannte Ketokörper, die den Appetit bis zum vollständigen Verlust der Nahrungsaufnahme weiter reduzieren. Als Folgekrankheiten entstehen insbesondere Leberschäden.

Eine Gabe größerer Mengen an Kohlenhydraten, insbesondere Zucker, verbietet sich wegen der Gefahr des Entstehens einer Pansenacidose durch selektive Wachstumsförderung säurebildender Pansenbakterien. Zuckerhaltige intravenöse Infusionen und Cortisoninjektionen sind die kostenintensiven tierärztlichen Standardtherapien zur Ketosebehandlung.

Der Erfindung liegt die Aufgabe zugrunde, ein Verfahren zur vorbeugenden Behandlung wenigstens einer der vorgenannten Gesundheitsstörungen bei kalbenden Kühen bereitzustellen.

Die Erfindung löst die Aufgabe durch ein Verfahren, bei dem den Kühen eine wirkstoffmischungenthaltenes Wasser zum Trinken angeboten wird, wobei die Wirkstoffmischung wenigstens einen trinkanregenden natürlichen oder naturidentischen Geschmacksstoff enthält, der einer chemischen Gruppe außerhalb der Gruppe der Zucker zugeordnet ist. Unter Zuckern werden in diesem Zusammenhang Monosaccheride und Disaccheride verstanden. Die Wirkstoffmischung im Sinne der Erfindung kann mehrere oder auch lediglich nur eine Komponente enthalten. Die Erfindung wird ferner durch eine Verwendung einer Wirkstoffmischung in dem vorgenannten Verfahren gelöst. Weitere vorteilhafte Ausgestaltungen der Erfindung sind in den Unteransprüchen beschrieben.

Durch das erfindungsgemäße Verfahren nehmen die zu behandelnde Kühe freiwillig größere Mengen Trinkwasser auf, da die Kühe das mit dem Geschmacksstoff angereicherte Trinkwasser gerne trinken. Hierdurch wird der Pansen der Kühe gefüllt und eine Verlagerung des Labmagens wirksam verhindert. Eine Zwangsverabreichung des Trinkwassers mit den sich daraus ergebenden unerwünschten Begleiterscheinungen erübrigt sich damit.

Der Geschmacksstoff wird bevorzugt als Bestandteil von Pflanzenteilen oder einem Pflanzenextrakt der Pflanzengattungen Stevien, Süßholzwurzel, Rosmarin, Enzianwurzel oder Wermut der Wirkstoffmischung zugesetzt. Es hat sich gezeigt, dass gerade Bestandteile oder Extrakte dieser Pflanzengattungen als Zugabe zum Trinkwasser geeignet sind, um die Wasseraufnahme der Kühe zu erhöhen.

Bevorzugt beträgt der Anteil der Pflanzenbestandteile und/oder Pflanzenextrakte der Stevien in dem den Kühen angebotenen Wasser 0,001 bis 50 Gewichtsprozent, insbesondere 0,005 bis 1 Gewichtsprozent und besonderes bevorzugt 0,001 bis 0,1 Gewichtsprozent. Mit Vorteil wird der Geschmacksstoff als Bestandteil von Pflanzenteilen oder einem Pflanzenextrakt der Stevienart "Stevia rebaudiana" der Wirkstoffmischung zugesetzt. Durch beide vorgenannte Merkmale, lässt sich unabhängig voneinander eine Erhöhung der von den Kühen aufgenommenen Trinkwassermenge erreichen.

Mit Vorteil handelt es sich beim Geschmacksstoff um in Stevien enthaltene Glykoside, insbesondere Steviolbiosid, Rebaudiosid A, Rebaudiosid B, Rebaudiosid C, Rebaudiosid E, Rebaudiosid F oder Dulcosid A. Es hat sich gezeigt, dass gerade der Zusatz dieser Glycoside zum Trinkwasser besonders geeignet ist, trinkanregend auf die Kühe zu wirken.

In einer besonders bevorzugten Ausgestaltung der Erfindung wird den Kühen das wirkstoffmischungenthaltende Wasser lauwarm und in einer Menge von 30 bis 80 Litern, insbesondere 45 bis 75 Litern und besonders bevorzugt 45 bis 55 Litern zu trinken angeboten. Zudem wird den kalbenden Kühen das wirkstoffmischungenthaltende Wasser mit Vorteil unmittelbar nach der Geburt und bevorzugt noch während die Kühe noch mit dem Abschlecken des Kalbes beschäftigt sind zum Trinken angeboten. Durch diese Maßnahmen wird der Pansen rechtzeitig vor einer Labmagenverlagerung gefüllt und beugt als Barriere einer Verlagerung vor.

In einer besonders bevorzugten Ausgestaltung der Erfindung enthält die Wirkstoffmischung zusätzlich Calcium. Durch die große Menge des aufgenommenen Trinkwassers ist es möglich, den Kühen eine ausreichende Menge Calcium freiwillig zu verabreichen. Das Calcium wirkt der Hypocalcaemie entgegen. Durch diese Ausgestaltung der Erfindung wird somit neben einer Verhinderung der Labmagenverlagerung auch einer Hypocalcaemie wirksam vorgebeugt.

Bevorzugt enthält die Wirkstoffmischung energiereiche Substanzen und Futtermittel, insbesondere glucoplastische Substanzen, wie Propionate und Propylenglykol. Durch die Aufnahme der sehr großen Menge Trinkwasser in Folge des Zusatzes der Geschmacksstoffe, nehmen die Tiere durch diese Ausgestaltung zusätzlich energiereiche Nahrungsmittel auf. Propionate und Propylenclykoll werden oft von den Tieren aufgrund ihres nachteiligen Geschmackes gemieden. Bedingt durch die große Verdünnung der Verbindungen in dem Trinkwasser und durch die dem Trinkwasser beigefügten Geschmacksstoffe ist jedoch auch eine Aufnahme größerer Mengen dieser glucoplastischen Substanzen möglich. Diese Ausgestaltung der Erfindung hat zur Folge, dass der eingangs beschriebenen Ketose kostengünstig und in einfacher Weise entgegengewirkt werden kann.

Mit Vorteil enthält die Wirkstoffmischung zudem Spurenelemente, Vitamine, Pro-Prä- und/oder Symbiotika sowie bevorzugt Tierarzneimittel. Diese Substanzen können den Tieren in einfacher Weise über das erfindungsgemäß angereicherte Trinkwasser zugeführt werden. Eine zusätzliche oder ergänzende Verabreichung dieser Verbindungen entfällt somit.

Nachfolgend wird ein Versuch der Anwendung des erfindungsgemäßen Verfahrens exemplarisch dargestellt.

### 1. Akzeptanzversuche verschiedener Kuhtränken im Holsteinzuchtbetrieb

| | | | |
|---|---|---|---|
| Betriebdaten im Jahr 2007: | | | |
| 150 Kühe | 10277 kg Milch | 4,02% Fett | 3,36% Eiweiss |
| Gleitender Herdendurchschnitt 2008: | | | |
| 182 Kühe | 10730 kg Milch | 4,08% Fett | 3,38 % Eiweiss |

| Produkte | Anzahl Kühe | durchschnittlich aufgenommene Menge Trinkwasser in l | Akzeptanz |
|---|---|---|---|
| A | 41 | 65 | sehr gut |
| B | 22 | 58 | sehr gut |
| C | 12 | 15 | mäßig |

| | | | |
|---|---|---|---|
| Produkt A: 30 g Pulver pro 10 l (60% Traubenzucker und Milchpulver) Wirk- und Füllstoffe: Calciumcarbonat ( 1g in 10 l Wasser) Produkt B: 20 g Pulver pro 10 l Wasser (6% Steviaextrakte) Wirk- und Füllstoffe: Calciumcarbonat (5 g in 10 l Wasser) Produkt C: Wasser ohne Zusatz von Wirkstoffen | | | |

### 2. Untersuchung zur Wirksamkeit verschiedener Kuhtränken im Holsteinzuchtbetrieb

| | | | |
|---|---|---|---|
| Betriebdaten im Jahr 2007: | | | |
| 150 Kühe | 10277 kg Milch | 4,02% Fett | 3,36% Eiweiss |
| Gleitender Herdendurchschnitt 2008: | | | |
| 182 Kühe | 10730 kg Milch | 4,08% Fett | 3,38 % Eiweiss |

| Produkte | Anzahl Kühe | aufgenommene Menge Trinkwasser | | Akzeptanz |
|---|---|---|---|---|
| | | Variation in l | Durchschnitt | |
| A | 41 | 65 | 40 - 100 l | sehr gut |
| B | 22 | 58 | 40 - 90 l | sehr gut |
| C | 12 | 15 | 0 - 60 l | mäßig |

| | | | | |
|---|---|---|---|---|
| Produkt A: 30 g Pulver pro 10 l (60% Traubenzucker und Milchpulver) Wirk- und Füllstoffe: Calciumcarbonat ( 1g in 10 l Wasser) Produkt B: 20 g Pulver pro 10 l Wasser (6% Steviaextrakte) Wirk- und Füllstoffe: Calciumcarbonat (5 g in 10 l Wasser) Produkt C: Wasser ohne Zusatz von Wirkstoffen | | | | |

Soweit das Produkt B vorhanden war, wurde es alternierend zu Produkt A eingesetzt.

### Ergebnisse Produkte A und B:

Bei gleicher Akzeptanz und Flüssigaufnahme ist bei Produkt B das Einbringen erheblich größerer, spezieller Wirkstoffmengen, wie bei Kühen erforderlich, unter wirtschaftlich vertretbaren Bedingungen möglich.

### Ergebnisse Produkt C:

9 Kühe nehmen lediglich 0-10 l Trinkwasser auf (Durchschnitt 5 l)
2 Kühe nehmen 40 l Trinkwasser auf
1 Kuh nimmt 60 l Trinkwasser auf

**Tabelle: Krankheitsinzidenz im Bestand**

| **Produkte** | **Milchfieber*** Anzahl Kühe | **Ketose** Anzahl Kühe | **Labmagenverlagerung** Anzahl Kühe |
|---|---|---|---|
| A | 8 | 1 | 1 |
| B | 2 | 0 | 1 |
| Bestands-% | 10 = 15,8 % | 1 = 1,5 % | 2 = 3,1 % |

| | | | |
|---|---|---|---|
| * Es wurde kein Festliegen beobachtet. Die Körperoberfläche der Kühe war kalt und sie machten einen matten Eindruck. | | | |

Milchfieber trat bei Gabe von Produkt A zu 19,5 % und bei Gabe von Produkt B zu 9 % auf. Bei einer Erhöhung der Calciumgabe ist eine weitere Reduktion des Auftretens von Milchfieber zu erwarten. In Beständen dieser Grössenordnung und dem angegebenen Leistungsniveau treten die aufgeführten Erkrankungen ohne Anwendung des erfindungsgemäßen Verfahrens deutlich häufiger auf.

### Gesamteindruck:

Im Vergleich zu den Vorjahren ohne erfindungsgemäße Trinkwassergabe zeigten die Kühe bei erhöhter Futteraufnahme eine verbesserte Vitalität.

## Patentansprüche

1. Verfahren zur vorbeugenden Behandlung von Gesundheitsstörungen bei kalbenden Kühen, bei dem den Kühen eine Wirkstoffmischung enthaltenes Wasser zum Trinken angeboten wird, wobei die Wirkstoffmischung wenigstens einen trinkanregenden, natürlichen oder naturidentischen Geschmacksstoff enthält, der einer chemischen Gruppe außerhalb der Gruppe der Zucker zugeordnet ist.

2. Verfahren nach Anspruch 1, **dadurch gekennzeichnet, daß** der Geschmacksstoff als Bestandteil von Pflanzenteilen oder einem Pflanzenextrakt der Pflanzengattungen Stevien, Süßholzwurzel, Rosmarien, Enzianwurzel oder Wermut der Wirkstoffmischung zugesetzt ist.

3. Verfahren nach Anspruch 2, **dadurch gekennzeichnet, daß** der Anteil der Pflanzenbestandteile und/oder Pflanzenextrakte der Stevien in dem den Kühen zum Trinken angebotenen Wasser 0,001 bis 50 Gew.-%, insbesondere 0,005 bis 1 Gew.-%, und bevorzugt 0,01 bis 0,1 Gew.-% beträgt.

4. Verfahren nach einem der vorhergehenden Ansprüche, **dadurch gekennzeichnet, daß** der Geschmacksstoff als Bestandteil von Pflanzenteilen oder einem Pflanzenextrakt der Stevienart Stevia rebaudiana der Wirkstoffmischung zugesetzt ist.

5. Verfahren nach einem der vorhergehenden Ansprüche, **dadurch gekennzeichnet, daß** der Geschmacksstoff ein in Stevien enthaltenes Glykosid, insbesondere Steviosid, Steviolbiosid, Rebaudiosid A, Rebaudiosid B, Rebaudiosid C, Rebaudiosid E, Rebaudiosid F oder Dulcosid A ist.

6. Verfahren nach einem der vorhergehenden Ansprüche, **dadurch gekennzeichnet, daß** den Kühen das Wirkstoffmischung enthaltene Wasser lauwarm und in einer Menge von 30 bis 80 Litern, insbesondere 45 bis 75 Litern und besonders bevorzugt 45 bis 55 Litern pro Tag zum Trinken angeboten wird.

7. Verfahren nach einem der vorhergehenden Ansprüche, **dadurch gekennzeichnet, daß** den kalbenden Kühen das Wirkstoffmischung enthaltene Wasser unmittelbar nach der Geburt zum Trinken angeboten wird.

8. Verfahren nach einem der vorhergehenden Ansprüche, **dadurch gekennzeichnet, daß** das den Kühen das Wirkstoffmischung enthaltene Wasser zum Trinken angeboten wird, noch während die Kühe mit dem Abschlecken des Kalbes beschäftigt sind.

9. Verfahren nach einem der vorhergehenden Ansprüche, **dadurch gekennzeichnet, daß** die Wirkstoffmischung Calcium enthält.

10. Verfahren nach einem der vorhergehenden Ansprüche, **dadurch gekennzeichnet, daß** die Wirkstoffmischung energiereiche Substanzen und Futtermittel enthält, insbesondere glucoplastische Substanzen, wie Propionate und Propylenglykol.

11. Verfahren nach einem der vorhergehenden Ansprüche, **dadurch gekennzeichnet, daß** die Wirkstoffmischung Spurenelemente, Mineralsalze, Vitamine, Pro-, Prae- und/oder Symbiotika enthält.

12. Verfahren nach einem der vorhergehenden Ansprüche, **dadurch gekennzeichnet, daß** die Wirkstoffmischung Tierarzneimittel enthält.

13. Verwendung einer Wirkstoffmischung nach einem der vorhergehenden Ansprüche in einem Verfahren nach einem der vorhergehenden Ansprüche.
